# EUROPEAN PATENT SPECIFICATION

(11) **EP 2 567 695 B1**
(45) Date of publication and mention of the grant of the patent: **26.10.2016**
(21) Application number: 11180754.1
(22) Date of filing: 09.09.2011
(51) Int. Cl.: A61K 31/122, A61K 31/352, A61K 31/355, A61K 31/385, A61K 31/4415, A61K 33/04, A61K 36/15, A61P 3/00, A61K 31/353, A61K 36/06

(54) **Antioxidant composition for reducing oxidative stress ascribable to treatment with hormonal contraceptives**
Antioxidative Zusammensetzung zur Verminderung von oxidativem Stress der der Behandlung mit hormonalen Kontrazeptiva zuzuschreiben ist
Composition antioxydante pour la réduction du stress oxydatif attribué au tratitement avec des contraceptifs hormonaux

(43) Date of publication of application: 13.03.2013
(73) Proprietor: Cornelli Consulting S.A.S. Di Umberto Cornelli & C., 20129 Milano (IT)
(72) Inventor: Cornelli, Umberto, 20129 MILANO (IT)
(74) Representative: Cattaneo, Elisabetta

(56) References cited:
- EP-A1- 2 364 697
- PINCEMAIL J ET AL: "Effect of different contraceptive methods on the oxidative stress status in women aged 40-48 years from the ELAN study in the province of Liege, Belgium", HUMAN REPRODUCTION, OXFORD UNIVERSITY PRESS, GB, vol. 22, no. 8, 1 August 2007 (2007-08-01) , pages 2335-2343, XP002596397, ISSN: 0268-1161, DOI: 10.1093/HUMREP/DEM146 [retrieved on 2007-06-20]
- CORNELLI U ET AL: "Bioavailability and antioxidant activity of some food supplements in men and women using the D-Roms test as a marker of oxidative stress", THE JOURNAL OF NUTRITION, WISTAR INSTITUTE OF ANATOMY AND BIOLOGY, vol. 131, no. 12, 1 December 2001 (2001-12-01), pages 3208-3211, XP002596399, ISSN: 0022-3166
- MOSCA L ET AL: "Modulation of apoptosis and improved redox metabolism with the use of a new antioxidant formula", BIOCHEMICAL PHARMACOLOGY, PERGAMON, OXFORD, GB, vol. 63, no. 7, 1 January 2002 (2002-01-01), pages 1305-1314, XP002230271, ISSN: 0006-2952, DOI: 10.1016/S0006-2952(02)00867-5

## Description

### FIELD OF THE INVENTION

The present invention relates to an antioxidant composition for reducing oxidative stress in subjects undergoing treatment with hormonal contraceptives. In particular, said composition has been found to be particularly suitable, since as well as having surprising efficacy, it is extremely well tolerated by the body.

### PRIOR ART

It was back in the 1950s that it was discovered that progesterone inhibits ovulation and that the problem of controlling pregnancy has aspects that are extremely complex and subtle, since in time side-effects were observed, such as thrombotic phenomena, the development of some types of tumours (i.e. of the breast), and conversely the reduction of other types of tumours (i.e. colorectal, ovarian). An element that was debated for a long time and was finally demonstrated is the presence of oxidative stress (OS) when contraceptive treatment (CT) is used **[1,2].** OS may in fact facilitate the onset of cardiovascular diseases and of thrombotic episodes and is present even if reduced dosages of oestrogens-progestogens are used, so that it seems to be closely linked to the contraceptive treatment itself. Although the reasons forming the basis of these circumstances have still not been fully elucidated, the principle of finding the best compromise between reduced dosage and efficacy remains valid.

In fact, there has been a transition from high dosages of progestogens to combinations of oestrogens with progestogens of various types, in an attempt to find the optimum ratio of benefit to risk. In this connection, systems are known for transdermal release, intrauterine release (intra uterine device, IUD), with subcutaneous or intramuscular deposition. Generally, the various types of contraceptives now available are prescribed on the basis of state of health and lifestyle (such as smoking).

However, even in healthy women, during the menstrual cycle an increase in phenomena of oxidation has been observed, and although they do not reach levels such as to be classified as OS, they nevertheless indicate an increase in oxidative phenomena corresponding to the proper phase of maturation of the ovum (oestrogenic phase) and the subsequent phase of possible implantation thereof (progestogenic phase).

For understanding the implications that give rise to the onset of OS, an examination of the hormonal aspects of the menstrual cycle is given below.

### Hormonal aspects connected with the normal menstrual cycle

The steroidal hormones associated with a woman's reproductive function are regulated by a central mechanism situated in the hypothalamic arcuate nucleus and in the anterior lobe of the pituitary gland, the neurons of which are correlated with the adrenal glands, the uterus and the ovary.

Briefly **[3],** the neurons of the arcuate nucleus release GnRH (gonadotropin-releasing hormone) hourly, timed and under noradrenergic stimulus; conversely, the opioid, dopaminergic and GABAergic neurons maintain an inhibitory state on said neurons.

It is therefore clear that it is the equilibrium of these "drivers" that determines the central vectorial stimulus in terms of frequency and amplitude (one peak/h with amplitude within about 2 mlU/ml of LH).

The GnRHs, via the so-called portal circulation (in the form of a spiral) wrapped around the pineal gland, then stimulate the anterior portion of the pineal gland for the pulsatile release of FSH (follicle-stimulating hormone) and LH (luteinizing hormone), which is thus associated with the release of GnRH; the FSH and LH regulate ovarian production of estradiol (E₂) and progesterone (Pg).

E₂ is the hormone of the so-called *proliferative phase;* this permits maturation of the ovarian follicle, stimulates cellular proliferation and cellular differentiation of the endometrium. In fact, both the cells of the mucosa and those of the endometrial vessels increase in number (and in volume); moreover, E₂ induces the production of receptors of Pg in the cells, so that the latter become more reactive to its presence when the corpus luteum begins to produce it.

Pg is the hormone of the *secretory phase,* i.e. preparatory to reception of the ovum and is derived from the corpus luteum that forms on the stroma of the emptied ovarian follicle. Its action limits the proliferative effect and stimulates vasal differentiation more definitely and the production of secretions that are more in keeping with implantation of the ovum (blastocyst). In this phase, progesterone also provides central stimulation of the inhibitory neurons (opioid, dopaminergic, GABAergic) of GnRH release.

Both E₂ and Pg, concomitantly with FSH, determine the modification of production of LH at the pituitary level; the effect of this modification is to reduce the frequency of the peaks of LH (one peak/3h instead of 1 peak/h) and to increase its amplitude (from 2 mlU/ml to 3-4 mlU/ml of LH).

This last aspect is functional in reducing the secretion of LH if implantation of the ovum does not occur, and then precipitating the levels of E₂ and Pg, concluding the cycle with the menstrual flow.

Conversely, in the case of implantation of the ovum, the chorionic gonadotropins (hGC), produced by the trophoblast and then by the placenta that is forming, stimulate the corpus luteum to continue producing functional E₂ and Pg for maintaining the conditions of the uterus for pregnancy. The placenta, once formed, will be self-sufficient in the production of both oestrogens.

There are then activities that are ancillary but are important for the purposes of reproduction by these oestrogens: in the fallopian tube, E₂ stimulates proliferation and differentiation while Pg reduces it; in the myometrium E₂ increases contractility while Pg reduces it; E₂ increases the amount and fluidity of the cervical mucus (for easier transit of the spermatozoa) while Pg reduces it, makes it more viscous and alters its consistency. However, one of the problems that is much debated is the action of these E₂ and Pg on oxidative stress. Dualism, by which one has an action opposite to the other, does not seem practicable, since both have a stimulating action on the use of cellular energy and metabolic induction even if for different tasks. Therefore, purely theoretically, both might be pro-oxidant, but the oxidative equilibrium depends on how much the stimuli of one or of the other permit the production of endogenous antioxidants. Therefore the state of the art on this particular subject is still in the phase of clarification and some salient aspects will be discussed below.

### Condition of OS during the normal menstrual cycle

Both the follicular cycle and the luteal cycle are divided schematically into an early phase and a late phase. This makes it possible for a course of the condition of OS described by some authors **[4]** to be differentiated schematically.

Basing the analysis on the local concentration (endometrial tissue) of GSH (reduced glutathione), of GSSG (oxidized glutathione), of GSHpx (glutathione peroxidase enzyme) and finally of MDA (malonyldialdehyde), it was possible to evaluate the change in oxidative conditions. These conditions were then correlated with the hormonal levels (estradiol, progesterone), of LH and FSH.

It emerges from these data that the peak phase of OS is observed in the middle phases of the cycle (i.e. late follicular and early luteal, or at the moment of maturation and possible implantation of the ovum. This phase is manifested by: a) an increase in production of GSHpx; b) a decrease in GSH; c) and an increase in GSSG; d) substantial stability of MDA.

This means that with increase in OS there is corresponding compensation so that the concentration of MDA (representation of lipid peroxidation) remains constant; in other words the system remains in equilibrium.

The peak of OS corresponds to the peak of oestrogens and the peak of LH, while the peak of progesterone appears to correspond to the phase of recovery from OS.

For this reason, it should be observed that GSHpx is the enzyme system that uses GSH as substrate, to permit reduction of hydroperoxides of various kinds (H₂O₂, or of lipid character such as ROOH etc.) forming H₂O.

GSHpx is of two types, GSHpx 1 which is present in the cytoplasm of all cells including the erythrocytes and GSHpx 3 which is present in the extracellular fluids, including plasma. The two types of enzymes, although both are formed from four identical subunits each containing a seleno-cysteine (Secys), in reality are different. In fact, the direct polyclonal antibodies to GSHpx 1 do not react with GSHpx 3. The GSPx 3 that is present in the plasma is synthesized primarily by the cells of the proximal renal tubules, but other cells are also able to synthesize it (liver, lung, heart, breast, intestine, brain, muscles) and to secrete it **[5].**

The reaction catalysed by GSH in relation to the hydroperoxides is as follows:

ROOH + 2 GSH = ROH +H₂O + GSSG

Therefore the increase in GSHpx corresponds in functional terms to an increase in oxidation of GSH; thus, in biological systems, its increase corresponds physiologically to a decrease in GSH with consequent increase in GSSG. This all signifies OS; therefore it is wrong to regard the GSHpx (of any type 1 or 3) as indicators of antioxidant capacity, whereas they should be regarded as an expression of an increased requirement for using GSH to cope with OS. However, if a clinical condition is characterized by reduced levels of GSHpx, this means that it will be more difficult to utilize GSH for purposes of coping with OS. This occurs, for example, in the menopause, both at endometrial and plasma level, but it also appears to be correlated with advanced age (typical condition of the menopause), and hence lower efficiency of the antioxidant system.

On analysing the system GSHpx, SOD (superoxide dismutase) and CAT (catalase) at the plasma level over the course of the menstrual cycle **[6],** it is observed that the erythrocyte GSHpx levels increase in the late follicular and early luteal phase relative to the other phases, while the levels of SOD and CAT remain substantially unchanged (see Table 1 below). Analysis of the correlations with the levels of E₂ indicate that the levels of GSHpx are temporally correlated (in extreme synthesis, increase of one occurs simultaneously with increase of the other).

The levels of FSH, LH, progesterone, testosterone, androstenedione do not show variations temporally correlated with those of erythrocyte GSHpx.

Accordingly, the isolatable action of E₂ is not antioxidant, but exactly the opposite **[7]** and corresponds to the cellular activation necessary for the increased energy requirement of the secretory phase.

The luteal phase, if the ovum has not been fertilized, is also characterized by an increase in cellular activity and by tissue proliferation, which precedes the end of the cycle with the menstrual flow that returns the uterine mucosa to the condition at the start of the cycle.

This phase is expressed in various ways in terms of final activity (changes in viscosity of the mucus, increase in the vascular component etc.) but in terms of oxidation it remains practically identical, since it is a proliferative phase.

This phase is dominated by the production of Pg, which is added to that of E₂, therefore, after the phase of maturation of the ovum, it has the highest expression of oestrogen-progestogen steroids.

**Table 1. Changes in the erythrocyte levels of enzymes relevant to oxidation**

| Variables | Phases of the cycle | | | |
|---|---|---|---|---|
| | Initial Follicular | Late Follicular | Initial Luteal | Late Luteal |
| GSH (lU/g Hb) | 15.2 ± 0.38 | 16.7 ± 0.23 | 16.3 ± 0.22 | 14.1 ± 0.31 |
| SOD (IU/100 µg Hb) | 1.3 ± 0.04 | 1.3 ± 0.02 | 1.2 ±0.03 | 1.3 ± 0.02 |
| CAT (lU/g Hb) | 18.6 ± 3.24 | 20.6 ± 2.28 | 19.6 ± 2.56 | 18.2 ± 3.12 |

A further characteristic of this period of the cycle is the consistent production of the NO^{•} radical. NO^{•} is an important mediator for vasodilatation, relaxation of the myometrium, antiplatelet action and the angiogenetic stimulation that develops via the VEGF (vascular endothelial growth factor), which in its turn stimulates NO synthetase **[8],** i.e. NO^{•} tries to maintain itself by auto-induction of NO synthetase. The latter, although developing through all the menstrual phases both in the eNOS (endothelial) and iNOS (induced) type, increases appreciably under progestogen stimulus **[9].**

Accompanying this increase, there is also locally an increase in SOD. SOD, effecting the dismutation of O₂^{•} (superoxide), prevents the reaction NO^{•} + O₂^{•} from forming the ion ONOO- (peroxynitrite) which, as well as having a high oxidant power, actually removes NO^{•}, limiting its availability. But all said available NO^{•} reacts with O₂^{•} (the reaction is between the two, is in absolute terms the fastest in the body (1 x 10⁻⁹ sec)) and finally generates OS.

The maintenance of vascular patency of the endometrium and its regular haematic distribution are also maintained by a particular peptide hormone, relaxin (RLX). RLX is a peptide with MW of 6 KDa and consists of two peptide chains stabilized both internally and between them with S-S bridges **[10].** In fact, it is a family of hormones (H₁, H₂, H₃), of which the circulating form H₂ is the most represented. RLX is primarily secreted by the corpus luteum and strategically prevents changes in endometrial flow, to avoid phenomena of ischaemia and reperfusion, during the proliferative phase of the cycle and then during possible pregnancy, which might trigger reactive processes with production of ROS and chemotaxis.

The action of RLX takes place by over-regulating iNOS and eNOS and angiogenesis in particular, but not only, in the endometrium; the action is exerted both on the arteriolar vessels and the venous vessels (therefore also on vessels without smooth muscle). The concentrations at which RLX is active are at the nanomolar level, and therefore obtainable during the true endometrial phases of implantation of the ovum and pregnancy. Angiogenetic action is also typical of RLX and develops through the release of VEGF as well as of bFGF (*basic fibroblast growth factor*).

An important aspect of the action of RLX is inhibition of endothelial adhesiveness of inflammatory white blood cells and platelet aggregation. This activity, combined with over-regulation of NO^{•} production, prevents development of the phenomenon of production of ROS and of chemotactic reaction typical of the condition of ischaemia/reperfusion.

Thus, overall, RLX has a very consistent influence on perfusion and blood distribution in the endometrium, where it maintains ideal flow conditions and exerts a typically anti-inflammatory and antithrombotic action.

### Action of oestrogens on the oxidation of lipoproteins

A final element to bear in mind is the action of oestrogens on the oxidation of lipoproteins. Many experiments conducted *in vitro* or *ex vivo* have demonstrated the antioxidant capacity of E₂, which exerts an action similar to that of α-tocopherol or of β-carotene on lipid peroxidation, more manifestly than its metabolites estradiol and estrone.

In actual fact, most of the experiments **[11-12]** were carried out using concentrations far higher than the physiological concentrations, at least of the order of 1-10 nmol/ml, corresponding to 273 and 2730 ng/mL respectively. These concentrations are attained just before the mid-cycle oestrogen peak and for limited periods of time; for this reason it is difficult to justify a protective antioxidant action of the LDLs **[13].**

However, in an experiment conducted in menopausal women, the administration of E₂ by the endoarterial route or as a transdermal patch (for 3 weeks) showed a delay in oxidation of LDLs (increase of the lag time or the oxidation time by Cu²⁺) of 11% and 16% respectively, with return to baseline conditions after suspending the treatment **[12].** However, there are strong doubts that the levels of E₂ obtained with the two types of administration were really the physiological levels, as they were determined at a single point; we only have to think of the haematic peak that is obtained with intraarterial administration before distribution has stabilized. Apart from this, no differences were observed in protective activity of LDLs between the two types of administration despite the notable differences in level (from 4 to 7 times higher with the patch).

Various studies have shown that for an antioxidant action to be observed (apart from the problem of levels) the E₂ must be brought into contact with the plasma, since if simply added to the LDLs, no activity is observed at all **[11].** This phenomenon was interpreted as a need for "activation" of E₂ by a plasma component, which was found to be LCAT (*lecithin-cholesterol acyltransferase*) which esterifies the E₂ with a fatty acid and it is only in these conditions that it can exert a protective action on lipoproteins. The esterification would take place on the carbon C17 leaving the hydroxyl group in C3 free; said ester would be inserted in the membrane of the lipoprotein with the hydrophilic moiety turned towards the outer surface and the lipophilic moiety inside the membrane **[14].** A similar configuration could theoretically protect the membrane from oxidation, evidently by oxidizing the ester of E₂, but there is still the question of whether this would be sufficient to protect an LDL.

The problem was then assessed from the stoichiometric standpoint.

The levels of E₂-esters in pre-menopausal women are barely determinable and the highest levels are found in the follicular fluid at a concentration of about 0.1 µmol/L **[15].** This all signifies that the ratio between lipoproteins (LDL, HDL, VLDL) is extremely high since only LDLs are present in the blood at a level of at least 0.6 µmol/L (corresponding to 1.6 g/L for a MW of 2.6 x 10⁶). Therefore the presence of E₂-esters in the lipoproteins is truly minimal (one molecule of E₂-ester to thousands of lipoproteins), incompatible with direct antioxidant action; therefore the possible mechanism of protection of LDLs may possibly be only of the enzymatic type (activation of the antioxidant enzymes present in the lipoproteins, such as paraoxonase, apoA I, apoJ, GSH px), but unknown at present.

Regarding the typology of the E₂-esters, those isolated from the follicular fluid are primarily of unsaturated nature to 96% (linolenates, arachidonates, palmitates) and only to 4% as saturated compounds (stearates) **[15].**

The E₂-esters are presumed to be one of the ways of keeping E₂ available as a reserve, since their presence is relatively consistent in omental and subcutaneous lipids (about 1 pmol/g of tissue) in pre-menopausal women, whereas they tend to decrease consistently during the menopause **[16].**

One thing to be considered concerning their presence on the lipoproteins is that in this way, in contrast to free oestrogens, they cannot be eliminated by the renal route and are transferred to the cells via the receptors for the LDLs. The free oestrogens can in fact, owing to their fat-solubility, easily cross the cell membrane less controllably.

Therefore, overall, the "strategic" significance of the E₂-esters is certainly not that of acting as direct antioxidants, but if necessary to be indirect antioxidants and to modulate the release of E₂, creating a reserve that can be activated as required. The mechanism of formation of the esters of steroidal hormones and their scant presence in the circulation but in protected form (lipoproteins) without bonds with albumins/globulins, nor with SBP (*steroid binding proteins*) or CBG (*corticosteroid binding globulins*) testify to the need to use them as reserve of steroids for prompt use if the need arises.

The E₂ are easily oxidizable, while the E₂-esters are less so.

The presence of MPO (myeloperoxidase) and of H₂O₂ (the latter is also secreted by the endothelial cells) causes oxidation of E₂, which behaves as a propagator of oxidation as well as inducing the production of MPO by neutrophils **[13].** However, the oxidative aspect of E₂ is very particular.

The behaviour seems similar to what occurs during stress **[17];** that is, oxidation of the LDLs which, in the circulation, are captured more easily by the hepatic receptors and kept under control and partly "muffled" by the muscular antioxidant systems. If, however, their quantity increases, because there is no adequate antioxidant system, they are subject to vascular uptake (subendothelial) triggering the atherosclerotic inflammatory process. In fact, in the subendothelium they would be subject to the action of MPO.

This accounts for the reduction of cholesterol and of LDL in pre-menopausal women, interpreted as greater efficiency of the hepatic receptors of LDLs and not increased uptake of LDL owing to their partially oxidized condition (it will be recalled that there is a minimum repairable oxidation limit, beyond which the macrophage reaction is triggered).

This oxidative equilibrium that is almost in balance can be defined as balanceable in physiological conditions of normal oestrogen/progestogen secretion (hence during a normal menstrual phase) but can be altered towards pro-oxidation when contraceptive therapy (CT) is used.

The phenomenon is understandable, since any oral administration of oestrogens/progestogens generates a blood peak of both (depending on the type of CT) which is not of the physiological level and therefore the possible "mild and controllable" oxidation becomes OS.

That this oxidative OS is generated by an increase in the oxidized level of LDLs, or by an increase in blood Cu levels, or by over-regulation of iNOs **[1]** or by all of them together has now been demonstrated **[1,2].** The problem that arises is that of controlling it, but to do all this it is first necessary to account for what happens in terms of OS in normal conditions, and then attempt to reduce it during treatment with contraceptives.

Pincemail et al. ('Effect of different contraceptive methods on the oxidative stress status in women aged 40 48 years from the ELAN study in the province of Liege, Belgium', Hum Reprod. 2007 Aug;22(8):2335-43) investigate the use of oral contraception (OC) in women, and concluded that the intake of OC significantly increases the lipid peroxidation in women aged 40-48 years. They state that this may represent a potential cardiovascular risk factor for these women.

Cornelli et al. ('Bioavailability and antioxidant activity of some food supplements in men and women using the D-Roms test as a marker of oxidative stress', J Nutr. 2001 Dec;131 (12):3208-11) refers to a method to monitor oxidative stress in serum. This test detects the derivatives of reactive oxygen metabolites (D-Roms). Hydroperoxides are converted into radicals that oxidize N,N-diethyl-para-phenylendiamine and that can be detected through spectrophotometric procedures as U.CARR. (Carratelli units). One U.CARR. corresponds to 0.8 mg/L hydrogen peroxide.

Mosca et al. ('Modulation of apoptosis and improved redox metabolism with the use of a new antioxidant formula', Biochem Pharmacol. 2002 Apr 1;63(7):1305-14) suggest that supplementation with antioxidant formulas can modulate the process of apoptosis under *in vivo* conditions. The clinical potential of the strategy they propose in the treatment of diseases with an elevated commitment to apoptosis is not yet explored.

There is therefore a need for a remedy that is effective and well tolerated by the body, that is able to reduce the oxidative stress ascribable to treatment with contraceptives of the hormonal type. The object of the present invention is thus to provide this remedy.

### SUMMARY OF THE INVENTION

Said object has been achieved by the antioxidant composition as claimed in claim 1, comprising, as active ingredients, selenium yeast, vitamin B6, alpha-lipoic acid, coenzyme Q10, alpha-tocopherol and proanthocyanidins.

According to another aspect, the present invention relates to the use of this composition for reducing oxidative stress ascribable to treatment with a hormonal contraceptive.

As will be clear from the following detailed description and from the working examples, provided for illustrative and non-limiting purposes, the composition of the invention surprisingly allows to reduce oxidative stress in subjects undergoing treatment with hormonal contraceptives by suitably combining antioxidant components with increased tolerability for the body.

### DETAILED DESCRIPTION OF THE INVENTION

The present invention therefore relates to an antioxidant composition comprising, as active components, selenium yeast, vitamin B6, alpha-lipoic acid, coenzyme Q10, alpha-tocopherol and proanthocyanidins. It has in fact been observed that the combination of these components allows, surprisingly and advantageously, to reduce the oxidative stress associated with taking of hormonal contraceptives. This formulation advantageously contains all types of antioxidants, i.e. systemic (selenium), within the cytoplasm (vitamin B6), mitochondrial (alpha-lipoic acid, coenzyme Q10), membrane (alpha-tocopherol), circulating (proanthocyanidins (PAN)).

The PAN are antioxidant plant metabolites belonging to the group of polyphenols. They occur in numerous plant species, but the most important source in the human diet is provided by cocoa seeds and grape seeds. However, preferably for the purposes of the present invention, the PAN are in the form of extract of *Pinus maritima,* also called *Pinus pinaster,* more preferably in the form of extract of bark of *Pinus maritima.*

Preferably this antioxidant composition comprises 10-70 wt.% of proanthocyanidins based on the total weight of the active components, where "total weight of the active components" means the sum of the weights of selenium yeast, vitamin B6, alpha-lipoic acid, coenzyme Q10, alpha-tocopherol and proanthocyanidins.

In these amounts, in fact the proanthocyanidins give maximum synergy with the other components, as will be clear also from the examples given herein below. More preferably, this antioxidant composition comprises 12-60 wt.% of proanthocyanidins based on the total weight of the active components.

Even more preferably, this antioxidant composition comprises 15-40 wt.% of proanthocyanidins based on the total weight of the active components.

According to a first preferred embodiment, this antioxidant composition comprises 58-59 wt.% of proanthocyanidins based on the total weight of the active components.

According to another preferred embodiment, this antioxidant composition comprises 38-39 wt.% of proanthocyanidins based on the total weight of the active components.

According to a further preferred embodiment, this antioxidant composition comprises 15-17 wt.% of proanthocyanidins based on the total weight of the active components.

Preferably, said antioxidant composition comprises 10-25 wt.% of selenium yeast, 0.5-5 wt.% of vitamin B6, 10-60 wt.% of alpha-lipoic acid, 1-10 wt.% of coenzyme Q10, 1-15 wt.% of alpha-tocopherol, based on the total weight of the active components.

More preferably, said antioxidant composition comprises 12-18 wt.% of selenium yeast, 1-4 wt.% of vitamin B6, 15-55 wt.% of alpha-lipoic acid, 2-6 wt.% of coenzyme Q10, 2-12 wt.% of alpha-tocopherol, based on the total weight of the active components.

According to a preferred embodiment, the antioxidant composition of the invention consists of selenium yeast, vitamin B6, alpha-lipoic acid, coenzyme Q10, alpha-tocopherol and proanthocyanidins, as active components, and optionally pharmaceutically acceptable excipients.

According to another preferred embodiment, said vitamin B6 is pyridoxine or a salt thereof. More preferably, it is pyridoxine hydrochloride.

According to another aspect, the present invention relates to an antioxidant composition for use as a medicament. In particular, said antioxidant composition is used for reducing oxidative stress ascribable to the treatment with a hormonal contraceptive. As will in fact be seen from the working examples given below, the composition of the invention shows a surprising synergistic effect resulting from suitable combination of the various components, which did not show any efficacy separately.

Preferably, said antioxidant composition is to be administered by the oral route. Preferably, said antioxidant composition is in the form of a unit dose comprising 15-130 mg of proanthocyanidins.

More preferably, said antioxidant composition is in the form of a unit dose comprising 22-110 mg of proanthocyanidins.

Even more preferably, said antioxidant composition is in the form of a unit dose comprising 28-75 mg of proanthocyanidins.

Preferably, said antioxidant composition is in the form of a unit dose comprising 15-40 mg of selenium yeast, 0.8-8 mg of vitamin B6, 15-115 mg of alpha-lipoic acid, 1.5-15 mg of coenzyme Q10, and 1.5-25 mg of alpha-tocopherol.

More preferably, said antioxidant composition is in the form of a unit dose comprising 18-30 mg of selenium yeast, 1-6 mg of vitamin B6, 20-105 mg of alpha-lipoic acid, 3-13 mg of coenzyme Q10, and 3-23 mg of alpha-tocopherol. Working examples of the present invention, provided for illustrative and non-limiting purposes, are presented herein below.

### EXAMPLES

### Example 1.

### Determination of antioxidant power of compositions of the invention by the PAT test (or SATp: Soluble Antioxidants Activity on plasma [18])

Ten healthy, eumenorrhoeic women volunteers were selected. The general characteristics of the volunteers are given below:

| Characteristics | Mean values ± SD* |
|---|---|
| Age (years) | 30.5 ± 5.66 |
| Weight (kg) | 68.5 ± 8.33 |
| Height (m) | 1.66 ± 0.07 |
| Body mass index (BMI kg/m²) | 24.7 ± 1.11 |

| | |
|---|---|
| * SD = standard deviation | |

The healthy volunteers were administered three different compositions of the invention (1A, 1B, 1C) with different dosages, as in Table 2.

**Table 2. Compositions investigated for activity in the PAT test**

| | Amount | Compositions | | |
|---|---|---|---|---|
| | | 1A | 1B | 1C |
| Proanthocyanidins (PAN)* | mg | 90 | 60 | 30 |
| Se yeast | mg | 25 | 25 | 25 |
| Vitamin B6 | mg | 5 | 4 | 2,4 |
| Alpha-lipoic acid | mg | 25 | 50 | 100 |
| Coenzyme Q10 | mg | 4 | 8 | 10 |
| Alpha-tocopherol | mg | 5 | 10 | 20 |

| | | | | |
|---|---|---|---|---|
| *Titrated at 90% of procyanidins | | | | |

The PAN were provided in the form of extracts of *Pinus maritima,* wherein said PAN were approx. 90 wt.%.

In the three compositions from 1A to 1C, the amount of PAN and of pyridoxine (in the form of hydrochloride) was gradually reduced and at the same time the amount of alpha-lipoic acid, vitamin E, selenium and coenzyme Q10 was gradually increased. In other words, the amount of soluble antioxidants was reduced and the amount of fat-soluble antioxidants was increased, whereas keeping the amount of selenium fixed at 50 mg (or 50% of RDA).

All the three compositions were administered to the volunteers over three successive days starting from the third day from the start of the menstrual cycle. No other therapy was allowed during this period of time. The experiment began in the morning, fasting since the previous evening, and only water was allowed for the period from 7 am to 13 am.

The compositions were always administered in the order A, B, C.

The PAT test (soluble antioxidants test for plasma) (distributor: H&D Parma-Italy) was performed by finger prick and collection in heparinized micro-test tube (0.15-0.2 mL of blood); four samples were taken: at baseline, and at 1 h, 3h and 5h (T1, T2, T3 respectively) after administration of each composition.

The differences between the values were calculated using the t-test and analysis of variance for orthogonal comparisons (ANOVA). The results are given in Table 3.

**Table 3. Values of PAT at different observation times following treatment with three different compositions of the invention. Mean values ± SD (standard deviation).**

| Time | Compositions | | | ANOVA |
|---|---|---|---|---|
| | 1A | 1B | 1C | |
| Baseline | 1719 ± 178.5 | 1734 ± 135.3 | 1695 ± 171.0 | A=B=C p > 0.05 |
| T1 (after 1 h) | 1756 ± 182.3 | 1818 ± 181.5^{a} | 1891 ± 132.0^{a} | A=B<C p< 0.05 |
| T2 (after 3 h) | 1894 ±163.6^{a} | 1959 ± 126.9^{a} | 2078 ± 171.2^{a} | A<B<C p < 0.05 |
| T3 (after 5 h) | 1697 ±147.6 | 1714 ± 115.2 | 2088 ± 205.0^{a} | A=B<C p < 0.05 |

| | | | | |
|---|---|---|---|---|
| ^{a} = t-test for interdependent data; TₙVs Baseline p< 0.05 | | | | |

It could be seen from the data that all the three compositions increased the antioxidant power of the plasma advantageously significantly at 3h after administration; moreover, compositions 1 B and 1C provided clear action even only 1 h after administration; composition 1C, after 1 h and 5 h respectively, unexpectedly showed significantly greater and more prolonged action with respect to the others, even though the content of PAN was far lower than in the other two. Thus, these observations demonstrated that all the compositions had antioxidant power, and so were bioavailable, but with pronounced efficacy of composition 1C, the action of which was more consistent and longer-lasting.

### Example 2.

### Evaluation of the activity of a contraceptive (CT) on the levels of OS and changes in OS following co-treatment with two different types of antioxidant products

For this test, 10 eumenorrhoeic women volunteers were selected, apparently healthy, without any other therapy in progress. The general characteristics were age < 35 years with BMI < 30 and without any current drug therapy or treatment with supplements, non-smokers, as shown in the following table.

| Characteristics | Mean values ± SD* |
|---|---|
| Age (years) | 29 ± 3.94 |
| Weight (kg) | 62.4 ± 4.22 |
| Height (m) | 1.60 ± 0.053 |
| Body mass index (BMI kg/m²) | 24.3 ± 1.75 |

| | |
|---|---|
| * SD = standard deviation | |

The volunteers followed three cycles of 21 days of hormonal contraceptive treatment (CT) with ethinylestradiol 50 µg combined with levonorgestrel 125 µg (Microgynon®: 21 days of treatment), said three cycles being named cycles A, B and C respectively.

During the first cycle (cycle A) the volunteers took the CT for 21 days combined with a placebo administered for 24 days; in the second cycle and in the third cycle they took CT combined instead with two different types of antioxidant products, named AR_{D} Stenovit^{®} (AOst) or AO2inv with cross-over: 5 volunteers with the sequence AOst/AO2inv and 5 volunteers with the sequence AO2inv/AOst with single blinding.

The respective formulations of AOst and AO2inv are given in Tables 4 and 5 and were taken if possible at bedtime for 24 days starting at the end of the menstrual cycle according to the scheme shown below.

| Cycle | Treatment |
|---|---|
| A | CT for 21 days Placebo for 24 days |
| B | CT for 21 days AOst for 24 days |
| C | CT for 21 days AO2inv for 24 days |

The placebo consisted of a vial containing only the excipients of the formulation shown in Table 4 (orange flavour, fructose, citric acid).

Throughout the test (three cycles of treatment with Mycrogynon® contraceptive pills), the volunteers attended the centre every three days, in the morning, between 8 o'clock and 9.30, fasting since the previous evening. The sample for evaluation of the d-ROMs test was taken from a finger; the blood was collected in an amount of 0.1-0.2 ml in microcuvettes containing heparin (10 µL of plasma is sufficient for the analysis of the d-ROMs test). The analyses were always carried out on the day of sampling, not more than two hours after obtaining the sample. Soon after the blood samples were taken, a standard breakfast was distributed. Otherwise no restrictions were imposed, except avoiding excessive food intake in the evening before sampling. In particular, they were asked to limit the consumption of chocolate, beyond what is customary, owing to the content of PAN in cocoa.

The formulation of the commercial product AR_{D} Stenovit^{®} (AOst) shown in Table 4 is known to possess antioxidant action, determined on the basis of the d-ROMs test when administered to healthy volunteers **[19]** in double-blind trials. In fact, this includes all four types of antioxidants, i.e. membrane antioxidants (beta-carotene, vitamin E, vitamin A), circulating antioxidants (Vit C, bioflavonoids), antioxidants within the cell (Coenzyme Q10 and pyridoxine) and systemic antioxidants (selenium and L-cysteine) which are the components of GSH and of GSHpx (glutathione and glutathione peroxidase respectively).

**Table 4. Composition of the antioxidant formulation (AOst).**

| Ingredients | Amount (mg) |
|---|---|
| Selenium yeast 0.2% | 24.00 |
| Vitamin C protected 97.5% | 30.77 |
| Bioflavonoids from *Citrus* strength 40% | 75.00 |
| Zinc pidolate | 25.00 |
| Coenzyme Q10 | 10.00 |
| L-cysteine hydrochloride | 12.61 |
| Vitamin E acetate strength 50% | 32.91 |
| Pyridoxine hydrochloride | 1.22 |
| Vitamin A acetate 50000 IU7g | 0.7 |
| Beta-carotene 10% | 0.5 |

The volunteers were administered AOst in the form of 1 vial of 10 mL two phases (to be mixed at the time of administration) simultaneously with CT pill.

The formulation AO2inv, as in Example 1C, was instead administered in capsules in an amount of 1 cps/day. The particularly distinctive elements of this formulation (see Table 5) were lipoic acid and the PAN combined with membrane antioxidants, (alpha-tocopherol), systemic (Se) and within the cell (pyridoxine and coenzyme Q10).

**Table 5. Composition of the invention (AO2inv)**

| Ingredients | measure | amount |
|---|---|---|
| Proanthocyanidins (PAN)* | mg | 30 |
| Se yeast | mg | 25 |
| Vitamin B6 | mg | 2.4 |
| Alpha-lipoic acid | mg | 100 |
| Coenzyme Q10 | mg | 10 |
| Alpha-tocopherol | mg | 20 |

| | | |
|---|---|---|
| * selenium yeast titrated as organic selenium at 0.2%, equal to 45 mcg of Se | | |

The mean values and standard deviations (SD) were determined for all the data; the differences between the baseline values and the various observation times were determined on the basis of the Student t-test for interdependent data. The comparisons between the values of the three cycles were determined on the basis of analysis of variance for orthogonal comparisons.

The results of the d-ROMs test are shown in Table 6.

Examination of the data showed that during the control cycle A, a consistent increase in mean values of d-ROMs was observed, above the normal values (which are <300 U.CARR.) which was evidence that OS was already present starting from the third day of therapy with CT, which continues until day 24 (t 24). All the cases analysed between t₃ and t₂₁ (period of taking the CT pill alone: cycle A) were clearly above the normal. After three days of suspension (t₂₄) of the treatment with CT, a recovery of the condition of OS was detected.

Next, in the second and third successive cycles (cycles B and C), daily administration of the formulations (AOST and AO2inv in cross-over) produced different results.

As could be seen from the mean values at different recording times (see Table 6), the treatment with AOst, which had been shown to be able to reduce the values of d-ROMs tests in healthy volunteers of both sexes **[19],** unexpectedly had no effect at all on the OS resulting from the treatment with hormonal contraceptive (CT).

**Table 6. Values of d-ROMs tests following CT with Microgynon^{®} for 21 days in healthy volunteers (mean ± SD)**

| | Time Days | t₁ | t₃ | t₆ | t₉ | t₁₂ | t₁₅ | t₁₈ | t₂₁ | t₂₄ |
|---|---|---|---|---|---|---|---|---|---|---|
| Cycle A (CT) | d-ROMS test U.CARR. | 287 ± 20.9 | 336^{a} ± 24.2 | 374^{a} ± 23.7 | 483^{a} ± 39.9 | 487^{a} ± 44.1 | 490^{a} ± 38.8 | 481^{a} ± 37.3 | 484^{a} ± 35.3 | 331^{a} ± 35.4 |
| | | ** | ** | ** | ** | ** | ** | ** | ** | * |
| Cycle B/C (CT + AOst) | d-ROMS test U.CARR. | ± 290 17.1 | 343^{a} ± 20.6 | 364^{a} ± 21.3 | 472^{a} ± 39.0 | 475^{a} ± 36.7 | 469^{a} ± 35.8 | 469^{a} ± 34.3 | 467^{a} ± 32.7 | 306^{a} ± 19.0 |
| | | ** | ** | ** | ** | ** | ** | ** | ** | * |
| Cycle B/C (CT + A02inv) | d-ROMS test U.CARR. | 293 ± 13.7 | 306^{a} ± 37.7 | 321^{a} ± 15.5 | 326^{a} ± 21.6 | 32₅a ± 21.1 | 326^{a} ± 19.2 | 313^{a} ± 12.4 | 312^{a} ± 21.8 | 284 ± 16.7 |
| | | ** | ** | ** | ** | ** | ** | ** | ** | * |
| ANOVA | Orthogonal comparison s | A=B=C | A=B>C | A=B>C | A=B>C | A=B>C | A=B>C | A=B>C | A=B>C | A=B>C |

| | | | | | | | | | | |
|---|---|---|---|---|---|---|---|---|---|---|
| ** treatment with CT and test products; * treatment only with the test products ^{a} = t-test for interdependent data p< 0.05 t₁ vs tₙ | | | | | | | | | | |

With the composition AO2inv, apart from the times t₁, all the values of d-ROMs tests were instead significantly lower than those obtained with the contraceptive treatment alone; that is, simultaneous treatment with the composition AO2inv had proved to be surprisingly significantly effective in reducing OS throughout the period of the cycle wherein the contraceptive drug was used.

These results were all the more surprising if considered that the use of the commercial antioxidant product AR_{D} Stenovit^{®}(AOst), of known and documented efficacy, using the same type of test for determining the state of OS, had proved to be completely unsatisfactory.

### Example 3.

### Evaluation of the synergistic effect showed by AO2inv relative to the action of only proanthocyanidins or of only physiological modulators with antioxidant action

This test was conducted on 24 apparently healthy volunteers. The purpose of the test was to verify whether the antioxidants contained in AO2inv, without proanthocyanidins, would still be active in reducing OS produced by the hormonal contraceptive with low oestrogen content (Yasmin®).

After a first period of 3 months of treatment with Yasmin®, three groups of volunteers were formed [A, B, C], each made up of 8 subjects selected on the basis of age, body weight and height in such a way that the three groups were as uniform as possible. All the volunteers had gone through at least a month of wash-out from any treatment apart from the contraceptive treatment. The characteristics of the volunteers are given below:

| Characteristics* | Group A | Group B | Group C |
|---|---|---|---|
| Age (years) | 30.8 ± 3.73 | 29.0 ± 3.66 | 29.9 ± 3.87 |
| Weight (kg) | 63.3 ± 2.92 | 64.6 ± 3.96 | 62.1 ± 3.44 |
| Height (m) | 1.63 ± 0.045 | 1.66 ± 0.063 | 1.64 ± 0.060 |
| Body mass index (BMI kg/m²) | 23.7 ± 0.96 | 23.6 ± 2.51 | 23.1 ± 1.29 |

| | | | |
|---|---|---|---|
| *All the general variables were analysed by ANOVA and the groups were found to be not significantly different (p > 0.05). | | | |

The three groups of volunteers were treated as follows:
- group A received daily treatment with AO2inv;
- group B was treated daily with only proanthocyanidins at a dose of 30 mg/day;
- group C was treated with the composition AO2inv (Table 5) but without proanthocyanidins.

The treatments began on the first day of taking the CT and were continued without interruption for two successive cycles of 24 days. Throughout the test, all the volunteers were allowed the use of coffee during the day according to their usual practice.

The treatments to be combined with CT were administered double-blind in indistinguishable hard capsules.

The values of d-ROMS tests were evaluated: a) at the end of a control cycle (between the nineteenth and the twenty-first day); b) in the second cycle of administration between the nineteenth and the twenty-first day of combined administration of CT products to be evaluated (therefore progressively between the forty-seventh and the forty-ninth day: see scheme given below)

| Days | Cycle | Operational details |
|---|---|---|
| From 1 to 21 | Control: only CT | Treatment with CT |
| From 19 to 21 | Control: only CT | Evaluation d-ROMs |
| From 27 to 48 | Combined therapy CT + MFs^{a} | Treatment with AO2inv or with PAN only or with AO2inv without PAN |
| From 46 to 48 | Combined therapy CT + MFs | Evaluation d-ROMs |

| | | |
|---|---|---|
| ^{a} MFs stands for Physiological Modulators and indicates that the treatment with CT is combined with antioxidants | | |

All the volunteers attended for the checks in the morning between 8 o'clock and 9.30, fasting since the previous evening. They were recommended to avoid excessive food intake during the evening before the blood tests, which were carried out according to the schedule given in Example 2.

The mean values and the standard deviations of the data were determined, and for comparisons between treatments, analysis of variance for orthogonal comparisons was applied, both at baseline and after 8 weeks of treatment.

The volunteers were given two boxes of 24 cps each, containing the respective formulations (AO2inv, PAN, or AO2inv without PAN), indistinguishable in appearance.

All the volunteers completed the study and the evaluation of compliance based on residual capsules was > 98%.

The results are given in Table 7.

**Table 7. Values of d-ROMs tests for the volunteers of groups A,B,C before and after treatment with antioxidant formulations. Mean values ± SD.**

| Period | Groups | | | ANOVA Orthogonal comparisons |
|---|---|---|---|---|
| | A | B | C | |
| Baseline (days from 19 to 21) | 490 ± 26.7 | 498 ± 27.8 | 480 ± 18.5 | A=B=C |
| after two cycles of combined treatment (days from 46 to 48) | 334^{a} ± 26.6 | 483 ± 33.4 | 461 ± 33.3 | B=C>A p< 0.05 |

| | | | | |
|---|---|---|---|---|
| ^{a} p<0.05 vs baseline | | | | |

Examination of the data clearly showed that the treatments with formulations of antioxidants only (group C) or with proanthocyanidins only (PAN) (group B) were not able to intervene minimally on OS. Therefore it was demonstrated that the significant efficacy in reduction of OS resulting from CT treatment was the result of the combination AO2inv (proanthocyanidins + Physiological Modulators with antioxidant action) giving an unexpected and surprising synergistic effect.

### Example 4.

Proanthocyanidins (PAN) of different origin were analysed, i.e. from cocoa seeds (titre at 75%), from *Pinus maritima* at two different types of yield (respectively from 65% to 75% and at 90%), from grape seeds (titre at 75%), to assess the similarity in terms of antioxidant action. The amounts administered to the apparently healthy volunteers were parameterized so that amounts equal to 30 mg of PAN were always administered. For the extracts of *Pinus maritima* between 65 and 75%, the average titre at 70% was considered.

The various preparations contained PAN, which were differentiated in terms of contents of oligomeric or polymeric anthocyanidins; that is, they were not identical, since the grade of PAN (from the dimers/pentamers of catechin and the oligomers from 8 to 24 formed from the epicatechin/catechin dimer) was different.

Therefore a different antioxidant bioavailability was to be expected.

Eight volunteers were observed, who were treated with all the products being analysed, according to a randomized protocol, by which, in each experimental session, all 4 experimental products were evaluated according to the scheme given below.

| Volunteer no. | Days | | | | | | |
|---|---|---|---|---|---|---|---|
| | 1 | 3-4 | 5 | 6-7 | 8 | 9-10 | 11 |
| | | * | | * | | * | |
| 1 | FA | | FB | | FC | | FD |
| 2 | FA | | FB | | FC | | FD |
| 3 | FB | | FA | | FD | | FC |
| 4 | FB | | FA | | FD | | FC |
| 5 | FC | | FD | | FA | | FB |
| 6 | FC | | FD | | FA | | FB |
| 7 | FD | | FC | | FB | | FA |
| 8 | FD | | FC | | FB | | FA |

| | | | | | | | |
|---|---|---|---|---|---|---|---|
| * days of wash-out | | | | | | | |

FA corresponds to fraction extracted from *Pinus maritima* or Pycnogenol® (titre at 65-75%);
FB corresponds to fraction extracted from *Pinus maritima* or pineol (titre at 90%)
FC corresponds to fraction extracted from cocoa seeds (titre at 75%)
FD corresponds to fraction extracted from grape seeds (titre at 75%)

The criteria for inclusion of the volunteers envisaged exclusively apparently healthy subjects who were not undergoing treatment with any type of drug or dietary supplement. Use of the contraceptive pill was regarded as a criterion for exclusion.

Each session of evaluation, with a duration of 5h, was followed by two days of wash-out without any treatment, in such a way that the evaluations of antioxidant activity did not fall on a Saturday or a Sunday.

The determinations of antioxidant power followed the scheme given in Example 1, except that the evaluations were only performed at 3h and 5h after administration. The conditions for sampling and the test for determination were identical to those in Example 1.

The data relating to the volunteers are presented in Table 8.

**Table 8. General characteristics of the healthy volunteers**

| Characteristics | Mean values ± SD* |
|---|---|
| Age (years) | 30.8 ± 3.73 |
| Weight (kg) | 63.3 ± 2.92 |
| Height (m) | 1.63 ± 0.045 |
| BMI (kg_{/}m²) | 23.7 ± 0.96 |

The results of the evaluation of plasma antioxidant activity (PAT) are presented in Table 9.

**Table 9. Values of PAT (Plasma Antioxidant Activity) following administration of PAN derived from various types of extracts; mean values ± SD**

| extract | Dose mg as PAN | Values of PAT µeq/L vitamin C | | |
|---|---|---|---|---|
| | | Baseline | 3h | 5h |
| FA | 30 | 1429 ± 126.5 | 1698 ± 174.1a | 1396 ±139.0 |
| FB | 30 | 1429 ± 126.5 | 1711 ± 175.6a | 1427 ±109.4 |
| FC | 30 | 1429 ± 126.5 | 1724 ± 133.7a | 1434 ±136.4 |
| FD | 30 | 1429 ± 126.5 | 1141 ± 128.4a | 1411 ± 135.4 |
| ANOVA | | Ns* | Ns | Ns |

| | | | | |
|---|---|---|---|---|
| ^{a} Student t-test for interdependent data p < 0.01 * Ns comparison between groups p > 0.05 | | | | |

It emerged from these data that, surprisingly, the various extracts of PAN did not show significant differences from one another and showed, as already mentioned, an increase in PAT (corresponding to µmol of Vitamin C/L) at 3h after administration with a return to baseline levels after 5h for all the extracts analysed, indicating substantial equivalence of the products in terms of circulating antioxidant power *in vivo.*

Therefore it was concluded that between the PAN extracted from various sources, there is substantial equivalence in terms of plasma antioxidant capacity, which can be regarded as a derivative of bioavailability.

The detailed description and the examples given above clearly show the advantages offered by the antioxidant composition according to the present invention. In particular, said composition has been shown to be particularly suitable for reducing oxidative stress in subjects undergoing treatment with hormonal contraceptives, advantageously being, moreover, extremely well tolerated by the body.

### References

1) De Groote D, Perrier d'Hauterive S, Pintiaux A et al. Effect of oral contraception with ethinylestradiol and drospirenone on oxidative stress in women 18-35 years old. Contraception 2009;80:187-193.
2) Pincemail J, Vanbelle S, Gaspard U et al. Effect of different contraceptive methods on the oxidative status in women aged 40-48 years from ELAN study in the province of Liege- Belgium. Human Reprod 2007;22:2335-2343.
3) Loose D, Stancel G. Estrogens and progestins in: Goodman & Gilman's The pharmacological basis of therapeutics 11th Ed 2006:1541-1553.
4) Serviddio G, Loverro G, Vicino M et al. Modulation of endometrial balance during the menstrual cycle: relation with sex hormones. J Clin Endocrinol Metab 2002;87:2843-2848.
5) Zachara BA, et al. Red blood cell and plasma glutathione peroxidase activities and selenium concentration in patients with chronic kidney disease: a review. Acta Biochim Pol 2006;53:663-677.
6) Massafra C, et al. Effects of estrogens and androgens on erythrocyte antioxidants superoxide dismutase, catalase and glutathione peroxidase activities during the menstrual cycle.J Endocrinol 2000;167:447-452.
7) Santanan N, Shern-Brewer, McClatchey R et al. Estradiol as an antioxidants: incompatible with its physiological concentrations and function. J Lipid Res 1998;39:2111-2118.
8) Fukumura D, Gohongi T, Kadambi A et al. Predominant role of endothelial nitric oxide synthase in vascular endothelial growth factor-induced angiogenesis and vascular permeability. Proc Soc Acad Sci USA 2001;98:2604-2609.
9) Cameron IT, Campbell S. Nitric oxide in the endometrium. Human Repr Update 1998;4:565-589.
10) Bani D. Relaxin as a natural agent for vascular health. Vasc Health Risk Manag 2008;4:515-524.
11) Sugioka K, Shimosegawa Y, Nakano M. Estrogerns as natural antioxidantss of membrane phospholipid peroxidation. FEBS 1987;210:37-39.
12) Sack MN, Rader DJ, O Cannon R. Oestrogens and inhibition of oxidation of low-density lipoproteins in postmenopausal women. Lancet 1994;343:269-270.
13) Chiang K, Parthasarathy S, Santanan N. Esrtogen, neutrophils oxidation. Life Sci 2004;75:2425-2438.
14) Larner JM, Pahuja SL, Shackleton CH et al. The isolation and characterization of estradiol-fatty esters in human ovarian follicular fluid. J Biol Chem 1993;268:13893-13899.
15) Tikkanen MJ, Vihma V, Jauhiainen M et al. Lipoproteins-associated estrogens. Cardiovasc Res 2001;56:184-188.
16) Larner JM, Shackleton CH, Roitman E et al. Measurement of estradiol-17-fatty acid esters in human tissue. J Clin Endocrinol Metab 1992;75:195-200.
17) Shern-Brewer R, Santanam N, Wetzstein C et al. Exercise and cardiovascular disease a new perspective. Arteriosc Thromb Vasc Biol 1998;18:1181-1187.
18) Cornelli U, Belcaro G, GM Nardi et al. Action of antioxidants complex on the antioxidants power of saliva. Pan Med 2010;52:69-73.
19) Cornelli U, Terranova R, Luca S, et al. Bioavailability and antioxidants activity of some food supplements in men and women using the D-Roms test as a marker of oxidative stress. J Nutr 2001;131:3208-3211.

## Claims

1. Antioxidant composition comprising, as active components, selenium yeast, vitamin B6, alpha-lipoic acid, coenzyme Q10, alpha-tocopherol and proanthocyanidins.

2. The antioxidant composition of claim 1, comprising 10-70 wt.% of proanthocyanidins based on the total weight of the active components.

3. The antioxidant composition of claim 2, comprising 12-60 wt.% of proanthocyanidins based on the total weight of the active components.

4. The antioxidant composition of claim 3, comprising 15-40 wt.% of proanthocyanidins based on the total weight of the active components.

5. The antioxidant composition of any one of claims 1-4, comprising 10-25 wt.% of selenium yeast, 0.5-5 wt.% of vitamin B6, 10-60 wt.% of alpha-lipoic acid, 1-10 wt.% of coenzyme Q10, 1-15 wt.% of alpha-tocopherol, based on the total weight of the active components.

6. The antioxidant composition of claim 5, comprising 12-18 wt.% of selenium yeast, 1-4 wt.% of vitamin B6, 15-55 wt.% of alpha-lipoic acid, 2-6 wt.% of coenzyme Q10, 2-12 wt.% of alpha-tocopherol, based on the total weight of the active components.

7. The antioxidant composition of any one of claims 1-6, wherein said proanthocyanidins are in the form of extract of *Pinus maritima.*

8. The antioxidant composition of claim 7, wherein said proanthocyanidins are in the form of extract of bark of *Pinus maritima.*

9. The antioxidant composition of any one of claims 1-8 for use as a medicament.

10. The antioxidant composition of any one of claims 1-8 for use in the treatment of oxidative stress ascribable to treatment with a hormonal contraceptive.

11. The antioxidant composition of any one of claims 1-10, in the form of a unit dose comprising 15-130 mg of proanthocyanidins.

12. The antioxidant composition of claim 11, in the form of a unit dose comprising 22-110 mg of proanthocyanidins.

13. The antioxidant composition of claim 12, in the form of a unit dose comprising 28-75 mg of proanthocyanidins.

14. The antioxidant composition of any one of claims 1-13, in the form of a unit dose comprising 15-40 mg of selenium yeast, 0.8-8 mg of vitamin B6, 15-115 mg of alpha-lipoic acid, 1.5-15 mg of coenzyme Q10, and 1.5-25 mg of alpha-tocopherol.

15. The antioxidant composition of claim 14, in the form of a unit dose comprising 18-30 mg of selenium yeast, 1-6 mg of vitamin B6, 20-105 mg of alpha-lipoic acid, 3-13 mg of coenzyme Q10, and 3-23 mg of alpha-tocopherol.

## Patentansprüche

1. Antioxidatives Gemisch, als aktive Komponenten beinhaltend:
Selenhefe, Vitamin B6, Alpha-Liponsäure, Koenzym Q10, Alpha-Tocopherol und Proanthocyanidine.

2. Antioxidatives Gemisch gemäß Anspruch 1, mit 10 - 70 Gew.-% Anteil von Proanthocyanidinen am Gesamtgewicht der aktiven Komponenten.

3. Antioxidatives Gemisch gemäß Anspruch 2, mit 12 - 60 Gew.-% Anteil von Proanthocyanidinen am Gesamtgewicht der aktiven Komponenten.

4. Antioxidatives Gemisch gemäß Anspruch 3, mit 15 - 40 Gew.-% Anteil von Proanthocyanidinen am Gesamtgewicht der aktiven Komponenten.

5. Antioxidatives Gemisch gemäß einem der Ansprüche 1 bis 4, mit 10 - 25 Gew.-% Anteil an Selenhefe, 0,5 - 5 Gew.-% an Vitamin B6, 10 - 60 Gew.-% an Alpha-Liponsäure, 1 - 10 Gew.-% an Koenzym Q10, 1 - 15 Gew.-% an Alpha-Tocopherol am Gesamtgewicht der aktiven Komponenten.

6. Antioxidatives Gemisch gemäß Anspruch 5, mit 12 - 18 Gew.-% Anteil an Selenhefe, 1 - 4 Gew.-% an Vitamin B6, 15 - 55 Gew.-% an Alpha-Liponsäure, 2 - 6 Gew.-% an Koenzym Q10, 2 - 12 Gew.-% an Alpha-Tocopherol am Gesamtgewicht der aktiven Komponenten.

7. Antioxidatives Gemisch gemäß einem der Ansprüche 1 bis 6, wobei die Proanthocyanidine in Form eines Extrakts von Pinus maritima vorliegen.

8. Antioxidatives Gemisch gemäß Anspruch 7, wobei die Proanthocyanidine in Form eines Extrakts der Rinde des Pinus maritima vorliegen.

9. Antioxidatives Gemisch gemäß einem der Ansprüche 1 bis 8, zum Gebrauch als Medikament.

10. Antioxidatives Gemisch gemäß einem der Ansprüche 1 bis 8 zum Gebrauch bei der Behandlung von oxidativem Stress, der der Behandlung mit hormonalen Kontrazeptiva zuzuschreiben ist.

11. Antioxidatives Gemisch gemäß einem der Ansprüche 1 bis 10, in Form einer Anteilsdosis mit 15 - 130 mg Proanthocyanidinen.

12. Antioxidatives Gemisch gemäß Anspruch 11, in Form einer Anteilsdosis mit 22 - 110 mg Proanthocyanidinen.

13. Antioxidatives Gemisch gemäß Anspruch 12, in Form einer Anteilsdosis mit 28 - 75 mg Proanthocyanidinen.

14. Antioxidatives Gemisch gemäß einem der Ansprüche 1 bis 13, in Form einer Anteilsdosis mit 15 - 40 mg Anteil an Selenhefe, 0,8 - 8 mg an Vitamin B6, 15 - 115 mg an Alpha-Liponsäure, 1,5 - 15 mg an Koenzym Q10 und 1,5 - 25 mg an Alpha-Tocopherol.

15. Antioxidatives Gemisch gemäß Anspruch 14, in Form einer Anteilsdosis mit 18 - 30 mg Anteil an Selenhefe, 1 - 6 mg an Vitamin B6, 20 - 105 mg an Alpha-Liponsäure, 3 - 13 mg an Koenzym Q10 und 3 - 23 mg an Alpha-Tocopherol.

## Revendications

1. Composition d'antioxydant comprenant, en tant que composants actifs, de la levure de sélénium, de la vitamine B6, de l'acide alpha-lipoïque, une coenzyme Q10, de l'alpha-tocophérol et des proanthocyanidines.

2. Composition d'antioxydant selon la revendication 1, comprenant 10 à 70 % en poids de proanthocyanidines sur la base du poids total des composants actifs.

3. Composition d'antioxydant selon la revendication 2, comprenant 12 à 60 % en poids de proanthocyanidines sur la base du poids total des composants actifs.

4. Composition d'antioxydant selon la revendication 3, comprenant 15 à 40 % en poids de proanthocyanidines sur la base du poids total des composants actifs.

5. Composition d'antioxydant selon l'une quelconque des revendications 1 à 4, comprenant 10 à 25 % en poids de levure de sélénium, 0,5 à 5 % en poids de vitamine B6, 10 à 60 % en poids d'acide alpha-lipoïque, 1 à 10 % en poids de coenzyme Q10, 1 à 15 % en poids d'alpha-tocophérol, sur la base du poids total des composants actifs.

6. Composition d'antioxydant selon la revendication 5, comprenant 12 à 18 % en poids de levure de sélénium, 1 à 4 % en poids de vitamine B6, 15 à 55 % en poids d'acide alpha-lipoïque, 2 à 6 % en poids de coenzyme Q10, 2 à 12 % en poids d'alpha-tocophérol, sur la base du poids total des composants actifs.

7. Composition d'antioxydant selon l'une quelconque des revendications 1 à 6, dans laquelle lesdites proanthocyanidines sont sous forme d'extrait de *Pinus maritima.*

8. Composition d'antioxydant selon la revendication 7, dans laquelle lesdites proanthocyanidines sont sous forme d'extrait d'écorce de *Pinus maritima.*

9. Composition d'antioxydant selon l'une quelconque des revendications 1 à 8 pour une utilisation en tant que médicament.

10. Composition d'antioxydant selon l'une quelconque des revendications 1 à 8, pour une utilisation dans le traitement de stress oxydatif attribuable à un traitement avec un contraceptif hormonal.

11. Composition d'antioxydant selon l'une quelconque des revendications 1 à 10, sous forme d'une dose unitaire comprenant 15 à 130 mg de proanthocyanidines.

12. Composition d'antioxydant selon la revendication 11, sous forme d'une dose unitaire comprenant 22 à 110 mg de proanthocyanidines.

13. Composition d'antioxydant selon la revendication 12, sous forme d'une dose unitaire comprenant 28 à 75 mg de proanthocyanidines.

14. Composition d'antioxydant selon l'une quelconque des revendications 1 à 13, sous forme d'une dose unitaire comprenant 15 à 40 mg de levure de sélénium, 0,8 à 8 mg de vitamine B6, 15 à 115 mg d'acide alpha-lipoïque, 1,5 à 15 mg de coenzyme Q10 et 1,5 à 25 mg d'alpha-tocophérol.

15. Composition d'antioxydant selon la revendication 14, sous forme d'une dose unitaire comprenant 18 à 30 mg de levure de sélénium, 1 à 6 mg de vitamine B6, 20 à 105 mg d'acide alpha-lipoïque, 3 à 13 mg de coenzyme Q10, et 3 à 23 mg d'alpha-tocophérol.
